# EUROPÄISCHE PATENTANMELDUNG

(11) **EP 1 208 795 A1**
(43) Veröffentlichungstag der Anmeldung: **29.05.2002**
(21) Anmeldenummer: 00440306.9
(22) Anmeldetag: 22.11.2000
(51) Int. Cl.: A61B 5/103

(54) **Verfahren zur in-vivo Bestimmung der Hauterschlaffung**

(71) Anmelder: Laboratoires Serobiologiques(Societe Anonyme), 54425 Pulnoy (FR)
(72) Erfinder: Perie, Gilles, 54000 Nancy (FR); Gillon, Véronique, 54270 Essey-les-Nancy (FR); Pauly, Gilles, 54000 Nancy (FR)
(74) Vertreter: Fabry, Bernd, Dr.

(57) **Zusammenfassung**

Die Erfindung betrifft ein Verfahren zur in-vivo Bestimmung der Hauterschlaffung, bei dem man auf eine erschlaffte Hautpartie eine Kraft oder Deformation einwirken lässt, anschließend die Relaxation misst und mit Hilfe der Abweichung der harmonischen Oberschwingung der Relaxationsantwort von der Linearität die gegenüber faltenfreier Haut überschüssige Hautfläche bestimmt.

## Beschreibung

### Stand der Technik

So wie in der Mehrzahl anderer fester Stoffe wirken auch in der menschlichen Haut Kräfte von innen ein; diesen Zustand bezeichnet man als inneren Stress. Bislang gibt es auf diesem Gebiet noch keine umfassenden Erkenntnisse. Die derzeit einzige Methode zur Bestimmung des inneren Stresszustandes der Haut besteht darin, zunächst den Ruhezustand zu untersuchen, die Haut dann in Streifen zu schneiden und festzustellen, ob eine Deformation eingetreten ist. Es liegt auf der Hand, dass dieses Verfahren für die Untersuchung der Wirksamkeit von kosmetischen Produkten in-vivo nicht geeignet ist.

Abweichend von dem Verhalten anderer Werkstoffe ist es wichtig zu wissen, dass die menschliche Haut keine Tendenz zeigt, innerem Stress durch Oberflächenvergrößerung entgegenzuwirken. Im Gegenteil zeigt gerade junge Haut eine straffe Oberfläche und der bekannte und erwünschte Effekt der Faltenbildung stellt lediglich das Ergebnis einer ständigen Relaxation der Hautschichten dar. Die kosmetische Chemie versucht diese Folgen der Hautalterung durch gezielte Verabreichung von Wirkstoffen entgegenzuwirken, in manchen Fällen bleibt aber nur noch die Möglichkeit, die infolge Faltenbildung nun überschüssigen Hautpartien chirurgisch zu entfernen; dies wird allgemein als "Lifting" bezeichnet.

Für den Chirurgen gibt es unterschiedliche Möglichkeiten, den Anteil der überschüssigen Hautpartien zu bestimmen, beispielsweise visuell (Visual Assessment) oder auf photographischem Wege (Bildanalyse). Es mangelt jedoch an einem echten in-vivo Verfahren. Die Aufgabe der vorliegenden Erfindung hat daher darin bestanden, gerade diesem Mangel abzuhelfen.

### Beschreibung der Erfindung

Gegenstand der Erfindung ist ein Verfahren zur in-vivo Bestimmung der Hauterschlaffung, bei dem man auf eine erschlaffte Hautpartie eine Kraft oder Deformation einwirken lässt, anschließend die Relaxation misst und mit Hilfe der Abweichung der harmonischen Oberschwingung der Relaxationsantwort von der Linearität die gegenüber faltenfreier Haut überschüssige Hautfläche bestimmt.

Mit Hilfe des geschilderten nicht-invasiven Verfahrens lässt sich das Ausmaß der Hautalterung im allgemeinen und speziell der Anteil durch Faltenbildung entstandener überschüssiger Hautfläche in-vivo bestimmen, was beispielsweise im Rahmen kosmetischer Operationen zu einer deutlichen Vereinfachung führt.

### Durchführung des Verfahrens

Die eigentliche Bestimmung der Relaxation erfolgt vorzugsweise elektrodynamisch. Dabei wird über eine Vorrichtung auf eine erschlaffte, insbesondere faltige Hautpartie (Gesicht, Oberarm, Oberschenkel etc.) eine vorzugsweise tangentiale (Deformations-) Kraft ausgeübt und die Relaxationsantwort mit Hilfe eines magnetischen Verdrängungsumwandlers (MDT, Magnetic Displacement Transducer) aufgezeichnet. Das Verfahren arbeitet dabei vornehmlich entweder unter Einsatz eines GBE (Gas Bearing Electrodynameter) oder eines BBE (Ball Bearing Elektrodynameter); ersteres wird beispielsweise in J.Soc.Cosmet.Chem. 36, 335 (1985) beschrieben, letzteres ist Gegenstand einer bislang unveröffentlichten Patentanmeldung der Anmelderin und besitzt eine Reihe von Vorteilen, nämlich beispielsweise eine bessere Übertragung der Signale und eine höhere Linearität von Kraft und Elongationssignal. Außerdem kann auf den Einsatz komprimierter Luft verzichtet werden. Alternative Möglichkeiten, eine Kraft auf die Hautfläche einwirken zu lassen, bestehen beispielsweise durch Einsatz eines Cutometers, bei dem die Krafteinwirkung durch Anlegen eines Vakuums von 100 bis 500 mbar entsteht, eines Twistometers, bei dem die Haut verdrillt wird, oder eines Indentometers. Diese Vorrichtung erlauben den Zusammenhang zwischen einwirkender Kraft und Deformation zeitbezogen zu beobachten. Dabei werden beispielsweise das Kraft/Elongationsverhältnis, der Deformationsgrad in Abhängigkeit der Dauer der einwirkenden Kraft oder die Grenze der cutanen Elastizität bestimmt.

### Signalerfassung und Analyse der Messergebnisse

Das Verfahren beruht wie schon erläutert darauf, dass auf eine Hautpartie eine (tangentiale) Kraft ausgeübt wird und die Deformation gemessen und ausgewertet wird. Folgt die Kraft über der Zeit einer Sinusfunktion und ist die Amplitude hinreichend groß, dann ist auch die nachfolgende Relaxationsschwingung der Haut notwendigerweise periodisch, wenngleich nur dann einer Sinusfunktion folgend, wenn die Deformation bzw. Verdrängung der Haut unter Krafteinwirkung der einwirkenden Kraft proportional ist; dies ist indes nur bei straffer, junger Haut der Fall. Ist die Haut im Gegensatz faltig, ist die Hautverdrängung der einwirkenden Kraft nicht proportional und die Relaxationsschwingung gegenüber einer Sinusschwingung gestört. Somit wird beruht das erfindungsgemäße Verfahren vereinfacht dargestellt darauf, dass man die Abweichung des Kraft/Elongationsverhältnis von der Linearität misst.

Die Signalerfassung kann analog (z.B. mit Hilfe von elektronischen Schaltungen, die aktive oder passive Filter zur Verstärkung enthalten) oder digital erfolgen. Die Signalverarbeitung geschieht dabei in gleicher Weise. Zur Auswertung der Signale eignet sich vorzugsweise die Fourier-Transformation, die mit Hilfe eines Spektrumanalysators vorgenommen werden kann (z.B. 3560C Portable Bruel & Kjaer Pulse). Zum Einsatz kann auch eine Fast Fourier Transformations-Software kommen. Die Amplitude der harmonischen Schwingungen wird mit Hilfe von Bandpass-Filtern (z.B. von der Firmen Bessel, Butterworth oder Chebyshev erhältlich) erhalten; anschließend wird das Signal rektifiziert. Schließlich kann man auch das Verfahren der Signalkorrelation anwenden. Dabei werden von einem Generator Signale der Frequenz sowie deren ungeraden Vielfachen erzeugt und eine Phasenverschiebung um 90° erzwungen. Die harmonischen Schwingungen und ihre Phasenverschiebung wird dann durch Signalkorrelation ermittelt (synchrone Rektifikation). Dabei stellt die einfachste Methode zur Korrelation von zwei Signalen die Entfernung der direkten Komponente mit Hilfe eines Hochpassfilters dar. Um aus diesen Signalen nun auf die überschüssige Hautfläche schließen zu können, geht man im allgemeinen von der ersten bis zehnten, vorzugsweise von der dritten, fünften, siebten und/oder neunten harmonischen Schwingung aus, die man als Länge ausdrückt und dann mit einem empirischen Faktor zwischen 2 und 10 multipliziert, welcher vom jeweiligen Hautzustand des Patienten abhängt.

Das erfindungsgemäße Verfahren eignet sich natürlich auch zum Nachweis der Wirksamkeit von kosmetischen Produkten oder Behandlungsverfahren, die die mechanischen Eigenschaften der Haut, der Schleimhäute oder des sub-cutanen Gewebes verbessern und so der Hauterschlaffung entgegenwirken, indem man die Menge erschlaffter Haut vor und nach deren Anwendung bestimmt. Bei den genannten kosmetischen Produkten kann es sich naturgemäß um sogenannte Anti-Ageingmittel, Antifaltenmittel, Aufbau- und Stärkungsmittel, Feuchtigkeitsspender, Straffungs- und Regenerationsmittel und dergleichen handeln. Bezüglich der Behandlungsverfahren sei auf die bekannten nicht-chirurgischen Liftingverfahren verwiesen.

### Beispiele

### Beispiel 1.

Zwei Probandengruppe bestehend aus jeweils 15 Personen im Alter von 18 bis 25 bzw. 65 bis 75 Jahren wurden untersucht. Bei den ausgewählten Hauptpartien handelte es sich um die Stirn, die Schläfen, die Oberarme sowie die Handrücken. Die Messung der Relaxation erfolgte mit Hilfe der BBE-Technik; die Auswertung, d.h. die Bestimmung überschüssiger Hautfläche in mm, erfolgte auf der Grundlage der 3. harmonischen Schwingung. Die Ergebnisse sind in Tabelle 1 zusammengefasst. Dabei wird deutlich, dass sich der Anteil überschüssiger Hautpartien im Laufe der Alterung durchschnittlich verdoppelt.

**Tabelle 1**

| **Überschüssige Hautpartien [mm]** | | | |
|---|---|---|---|
| **Hautpartie** | **Probanden jung** | **Probanden alt** | **Faktor Differenz** |
| Stirn | 0,924 | 2,439 | 2,6 |
| Schläfen | 2,5 | 3,158 | 1,26 |
| Oberarme | 1,263 | 2,478 | 2 |
| Handrücken | 1,186 | 2,713 | 2,3 |

### Beispiel 2.

In gleicher Weise wurde die Wirksamkeit eines kosmetischen Wirkstoffs (Hiniscin PW LS, hydrolysierte Hibiscusproteine, Labratoires Sérobiologiques, S.A./ Nancy) gegen eine Placebocreme auf die Hauterschlaffung getestet. Die Testgruppe bestand aus 9 weiblichen Probanden im Alter von 46 bis 65 Jahren. Die Anwendung erfolgte im Stirnbereich jeweils morgens und abends über einen Zeitraum von 6 Wochen. Die Ergebnisse sind in Tabelle 2 zusammengefasst.

**Tabelle 2**

| **Überschüssige Hautpartien [mm]** | | | |
|---|---|---|---|
| **Hautpartie** | **Placebo** | **mit Wirkstoff** | **Faktor Differenz** |
| zu Beginn | 2,246 | 2,188 | - 0,290 |
| nach 6 Wochen | 1,956 | 1,717 | - 0,471 |

## Patentansprüche

1. Verfahren zur in-vivo Bestimmung der Hauterschlaffung, bei dem man auf eine erschlaffte Hautpartie eine Kraft oder Deformation einwirken lässt, anschließend die Relaxation misst und mit Hilfe der Abweichung der harmonischen Oberschwingung der Relaxationsantwort von der Linearität die gegenüber faltenfreier Haut überschüssige Hautfläche bestimmt.

2. Verfahren nach Anspruch 1, **dadurch gekennzeichnet, dass** man auf die Hautpartie eine tangentiale Kraft oder Deformation einwirken lässt.

3. Verfahren nach den Ansprüchen 1 und/oder 2, **dadurch gekennzeichnet, dass** man die Relaxation elektrodynamisch misst.

4. Verfahren nach mindestens einem der Ansprüche 1 bis 3, **dadurch gekennzeichnet, dass** man die Abweichung des Kraft/Elongationsverhältnis von der Linearität misst.

5. Verfahren nach mindestens einem der Ansprüche 1 bis 4, **dadurch gekennzeichnet, dass** man zur Berechnung der überschüssigen Hautfläche die 1. bis 10. harmonische Relaxationsschwingung heranzieht.
